# EUROPÄISCHE PATENTSCHRIFT

(11) **EP 3 181 546 B1**
(45) Veröffentlichungstag und Bekanntmachung des Hinweises auf die Patenterteilung: **02.05.2018**
(21) Anmeldenummer: 15200490.9
(22) Anmeldetag: 16.12.2015
(51) Int. Cl.: C07C 67/38, C07C 69/34, C07C 69/44, C07C 69/612, C07C 69/42

(54) **VERFAHREN ZUR DOPPELTEN CARBONYLIERUNG VON ALLYLALKOHOLEN ZU ENTSPRECHENDEN DIESTERN**
METHOD OF DOUBLE CARBONYLATION OF ALLYLIC ALCOHOLS IN ORDER TO FORM CORRESPONDING DIESTERS
PROCEDE DE CARBONYLATION DOUBLE D'ALCOOLS ALLYLIQUES EN DI-ESTERS CORRESPONDANTS

(43) Veröffentlichungstag der Anmeldung: 21.06.2017
(73) Patentinhaber: Evonik Degussa GmbH, 45128 Essen (DE)
(72) Erfinder: HAOQUAN, Li, Xiaolan Town, 528415 Zhongshan (CN); LIU, Jie, Hunan, 412100 (CN); BELLER, Matthias, 18211 Ostseebad Nienhagen (DE); JACKSTELL, Ralf, 27478 Cuxhaven Altenwalde (DE); FRANKE, Robert, 45772 Marl (DE); DYBALLA, Katrin Marie, 45657 Recklinghausen (DE)

(56) Entgegenhaltungen:
- EP-A2- 0 146 859
- JOHN F. KNIFTON: "Syngas reactions", JOURNAL OF ORGANOMETALLIC CHEMISTRY., Bd. 188, Nr. 2, 1. April 1980 (1980-04-01) , Seiten 223-236, XP055272822, CH ISSN: 0022-328X, DOI: 10.1016/S0022-328X(00)82815-6
- QIANG LIU ET AL: "Domino Catalysis: Palladium-Catalyzed Carbonylation of Allylic Alcohols to [beta],[gamma]-Unsaturated Esters", ANGEWANDTE CHEMIE INTERNATIONAL EDITION, Bd. 52, Nr. 31, 29. Juli 2013 (2013-07-29) , Seiten 8064-8068, XP055273131, DE ISSN: 1433-7851, DOI: 10.1002/anie.201303850
- KENJI ITOH ET AL: "Palladium-catalyzed carbonylation of allyl alcohols in the presence of lithium chloride and titanium(IV) isopropoxide", JOURNAL OF MOLECULAR CATALYSIS, Bd. 75, Nr. 2, 1. September 1992 (1992-09-01), Seiten 117-122, XP055273137, NL ISSN: 0304-5102, DOI: 10.1016/0304-5102(92)80112-T

## Beschreibung

Die Erfindung betrifft ein Verfahren zur doppelten Carbonylierung von Allylalkoholen zu den entsprechenden Diestern, wobei ein linearer oder verzweigter Allylalkohol mit einem linearen oder verzweigtem Alkanol (Alkohol) unter Zuführung von CO und in Gegenwart eines katalytischen Systems aus einem Palladiumkomplex und mindestens einem organischen Phosphorliganden sowie in Anwesenheit eines Halogenwasserstoffs ausgewählt aus HCl, HBr oder HJ zur Reaktion gebracht wird.

Es existiert eine Vielzahl von Methoden, die Bedeutung für die Synthese spezieller Ester besitzen. Insbesondere Allylalkohol ist eine nachhaltige organische Verbindung, die bereits eine breite Anwendung gefunden hat. So wurde z.B. die direkte Carbonylierung der CO-Einheit um β,γ-ungesättigte Carbonsäure-Derivate zu synthetisieren, zuerst 1964 von Tsuji untersucht [J. Tsuji, J. Kiji, S. Imamura, M. Morikawa, J. Am. Chem. Soc. 1964, 86, 4350-4353]. Hier wurden verschiedene Allylverbindungen wie Allyl-Chlorid, -Bromid, Allylether und Allylalkohol nacheinander in die entsprechende β,γ-ungesättigten Carbonsäureestern in Gegenwart von Palladiumchlorid unter CO-Druck überführt. Allylalhohol wird in der Industrie vor allem auch zur Herstellung von 1,4-Butandiol durch Hydrierung ihres Hydroformylierungsproduktes eingesetzt [a) J. C. Pittman, W. Honnick, J. Org. Chem. 1980, 45, 2132-2139; b) D. ARLT,(UMICORE AG & CO. KG), WO 2012/163831, 2012**;** c) W. S. Dubner, W. P.-s. Shum,(ARCO Chemical Technology, L.P.), United States, US 6225509 B1, 2001**;** d) M. Matsumoto, M. Tamura,(Kuraray Co., Ltd.), United States, US 4238419 A, 1980**;** e) M. Matsumoto, S. Miura, K. Kikuchi, M. Tamura, H. Kojima, K. Koga, S. Yamashita,(Kuraray Company, Ltd. Daicel Chemical Industries, Ltd.), United States, US 4567305 A, 1986**;** f) D. F. White, W. S. Dubner,(Lyondell Chemical Technology, L.P. (Greenville, DE, US)), United States, US 7271295 B1, 2007**;** g) J. G. Zajacek, W. P. Shum,(Arco Chemical Technology, L.P.), United States, US 6127584 B1, 2000].
1980 wurde ein Katalysatorsystem in Kombination von Palladium Halogenid wie PdCl₂, PdBr₂, eines Phosphinliganden, beispielsweise PPh₃, P(p-Tol)₃ veröffentlicht und Cokatalysatoren wie CaCl₂ und CoCl₂ veröffentlicht für die Carbonylierung von allylischen Verbindungen, wie z.B. Allylchlorid, -bromid und Allylalkohole [J. F. Knifton, J. Organomet. Chem. 1980, 188, 223-236]. 1992 wurde eine ähnliche Arbeit von Miura und Mitarbeitern in Gegenwart von Lithiumchlorid und Titanisopropoxid als Reaktionspromotor als ein weiteres Beispiel der Carbonylierung von Allylalkohol beschrieben [K. Itoh, N. Hamaguchi, M. Miura, M. Nomura, J. Mol. Catal. 1992, 75, 117-122]. 1997 wurde über die Reaktion in Gegenwart von Phenol berichtet [T. Satoh, M. Ikeda, Y. Kushino, M. Miura, M. Nomura, J. Org. Chem. 1997, 62, 2662-2664]. 2013 wurde ein allgemeineres und praktischeres Verfahren für die Alkoxycarbonylierung von Allylalkohol durch Beller et al. (Gleichung 1) beschrieben [Q. Liu, L. Wu, H. Jiao, X. Fang, R. Jackstell, M. Beller, Angew. Chem. Int. Ed. 2013, 52, 8064-8068]. Das Katalysatorsystem besteht aus Pd (OAc)₂, Phosphin-Liganden, wie beispielsweise Xantphos oder BuPAd₂ und Trifluoressigsäure. Es wurde ein breites Spektrum von Substratspektren vorgestellt sowie mechanistische Studien durchgeführt. In allen berichteten Fällen wurde die ungesättigte C = C-Bindung nach der Reaktion zurückerhalten.

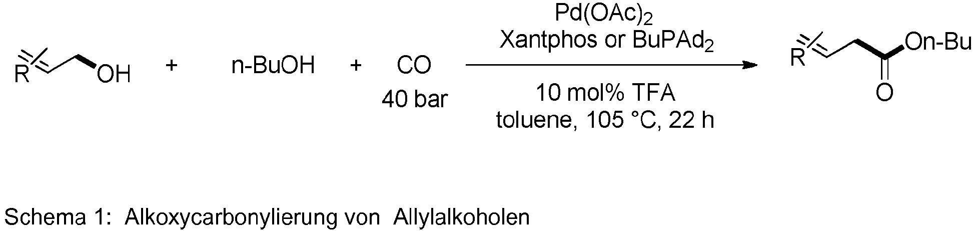

Zusammenfassend ist festzustellen, dass die selektive Alkoxycarbonylierung der olefinischen Einheit von Allylalkoholen eine Herausforderung bleibt und dass keine Verfahren zur Carbonylierung von Allylalkoholen bekannt sind, die zu Diestern führen. Diester sind bekanntermaßen wichtige Monomere in der Polymerindustrie zur Synthese von Polyestern und Polyamiden über Kondensationsreaktionen wie z. B. Poly(ethylen)adipat und Nylon-66. Die Diester monomerer Verbindungen kann man auch als Vorstufen bei der Herstellung von Farbstoffen, Arzneimitteln und Agrikulturprodukten verwenden.

Es besteht daher ein großer Bedarf an Carbonsäurediestern. Der Erfindung liegt deshalb die Aufgabe zugrunde, eine effektive und kostengünstige Methode zur Synthese von Diestern zu finden.

Die Aufgabe wird durch ein Verfahren gemäß Anspruch 1 gelöst. Die Unteransprüche stellen bevorzugte Verfahrensvarianten dar. Es wird eine neue Methode zur Synthese von Diestern über eine doppelte Carbonylierung von Allylalkoholen beschrieben. Die erfindungsgemäß hergestellten Produkte - die Diester - liegen vorzugsweise als Isomerengemische vor.

Das Verfahren zur doppelten Carbonylierung von Allylalkoholen zu Diestern ist dadurch gekennzeichnet, dass ein linearer oder verzweigter Allylalkohol mit einem linearen oder verzweigtem Alkanol unter Zuführung von CO und in Gegenwart eines katalytischen Systems aus einem Palladiumkomplex und mindestens einem organischen Phosphorliganden sowie in Anwesenheit eines Halogenwasserstoffs ausgewählt aus HCl, HBr oder HJ zur Reaktion gebracht wird. Bevorzugte Halogenwasserstoffe sind HCl und HBr. Ganz besonders bevorzugt ist der Einsatz von HCl.

Allylalkohole sind vorzugsweise Verbindungen der allgemeinen Formel (1) 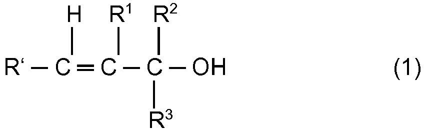 wobei
R¹, R² und R³ unabhängig voneinander Wasserstoff oder einen C₁ bis C₁₀ Alkylrest bedeuten und
R' für Wasserstoff, oder einen gesättigten oder ungesättigten, verzweigten oder unverzweigten aliphatischen, cycloaliphatischen oder cycloaliphatisch-aliphatischen Kohlenwasserstoffrest mit bis zu 12 C-Atomen, in dem C-C-Bindungen durch Sauerstoff oder die -O-CO-Gruppe unterbrochen sein können, oder einen Phenylrest steht, wobei der Phenylrest wie folgt substituiert sein kann: C₁- bis C₁₀-Alkyl- oder C₁- bis C₁₀-Alkoxy-Gruppen.

Alkyl bedeutet bevorzugt einen verzweigten oder unverzweigten Rest mit 1 bis 6 Kohlenstoffatomen. Alkylgruppen sind z.B. Methyl, Ethyl, Propyl, Isopropyl, 1-Butyl, 2- Butyl, 1-Pentyl, 1-Hexyl.

Die erfindungsgemäß eingesetzten Alkohole können sowohl primäre als auch sekundäre Alkohole eingesetzt werden. Es können sowohl aliphatische, cycloaliphatische, aromatische als auch araliphatische Alkohole benutzt werden, vorzugsweise finden aliphatische und araliphatische Alkohole Anwendung. Im Allgemeinen werden Alkohole ROH im erfindungsgemäßen Verfahren verwendet, in denen der Rest R eine C₁- bis C₁₀-Alkyl-, eine C₁- bis C₂₀-Cycloalkyl- oder eine C₇- bis C₁₁-Aralkylgruppe ist.

Phenyl für R' und R in ROH können gegebenenfalls mit Substituenten wie C₁- bis C₁₀-Alkyl- oder C₁- bis C₁₀-Alkoxy-Gruppen substituiert sein.
Es werden vorzugsweise Alkohole ROH mit unsubstituierten Resten R verwendet. Selbstverständlich können auch Alkohole mit einer höheren Anzahl an Kohlenstoffatomen eingesetzt werden. Es werden vor allem niedere Alkanole (C₁ bis C₆) bevorzugt verwendet.

Beispiele für aliphatische Alkohole sind z. B. Methanol, Ethanol, 1-Propanol, 2-Propanol, C₄-Alkohole, z. B. 1-Butanol, 2-Butanol oder Isobutylalkohol, C₅-Alkohole, z. B. 1-Pentanol, Isoamylalkohol, oder 2-Pentanol, C₆-Alkohole, z. B. 1-Hexanol, 2-Methyl-1-pentanol, 3-Methyl-1-pentanol, 2,2-Dimethyl-1-butanol, 2-Ethyl-1-butanol, 4-Ethyl-1-pentanol, 2-Hexanol, 3-Hexanol, 3-Methyl-2-pentanol, 2,3-Dimethyl-2-butanol, 2-Methyl-3-pentanol, 3-Methyl-3-pentanol, 4-Methyl-2-pentanol, 2-Methyl-2-pentanol, C₇-Alkohole, z. B. n-Heptylalkohol, 2-Methyl-1-hexylalkohol, 3-Methyl-1-hexylalkohol, 4-Methyl-1-hexylalkohol, 5-Methyl-1-hexylalkohol, 2-Ethyl-1-pentanol, 3-Ethyl-1-pentanol, 2,2-Di-methyl-1-pentanol, 3,3-Dimethyl-1-pentanol, 4,4-Dimethyl-1-pentanol, 2,3-Dimethyl-1-pentanol, 2,4-Dimethyl-1-pentanol, 3,4-Dimethyl-1-pentanol, C₈-Alkohole, z. B. 1-Octanol, 2-Methyl-1-heptanol, 3-Methyl-1-heptanol, 4-Methyl-1-heptanol, 5-Methyl-1-heptanol, 2-Octanol, 3-Octanol, 4-Octanol, 2-Methyl-2-heptanol, 3-Methyl-2-heptanol, 4-Methyl-2-heptanol, 5-Methyl-2-heptanol, 6-Methyl-2-heptanol, 2-Methyl-3-heptanol oder 3-Methyl-3-heptanol, und C₉-Alkohole, z. B. 1-Nonanol. Beispiele für die alicyclischen Alkohole mit 4 oder mehr Kohlenstoffatomen umfassen alicyclische Alkohole mit 4 bis 12 Kohlenstoffatomen, wie z. B. Cyclopentanol, Cyclohexanol oder Cyclooctanol.
Als C₇- bis C₁₁-Aralkylgruppe wird vorzugsweise die Benzylgruppe eingesetzt.

In einer Variante des Verfahrens wird die Reaktion in flüssiger Phase bei einer Temperatur von 70 bis 250 °C, vorzugsweise bei 80 bis 180 °C, besonders bevorzugt bei Temperaturen von 80 bis 150 °C durchgeführt.

Die Reaktion findet bevorzugt unter einem Druck von 2 bis 100 bar statt. Vorzugsweise wird die Reaktion bei einem Druck von 5 bis 50 bar durchgeführt.

In einer Variante des Verfahren wird der Palladiumkomplex in-situ ausgehend von einem Vorkomplex gebildet wird, wobei als Palladiumquelle Palladium-enthaltende Salze und Komplexe als Vorstufe verwendet werden. Die Palladiumverbindungen können in unterschiedlichen Oxidationsstufen vorliegen, dabei sind vorteilhafterweise die Stufen 0 bis +IV umfasst. Vorzugsweise ist der Palladiumkatalysator ausgewählt aus der Gruppe enthaltend Pd-Acetat, Pd-Acetylacetonat, Pd-Halogenide und Pd-Halogen-1,5-cyclooctadiene, Pd-Nitrate, Pd-Oxid und Diammoniumhexachloropalladat.
Eine besonders bevorzugte Vorstufe ist Pd-Acetylacetonat.

Die bevorzugten Phosphinliganden L weisen eine mono- oder bidentate Struktur auf. So werden z.B. die Liganden L1 bis L12 besonders vorteilhaft im erfindungsgemäßen Verfahren eingesetzt:
L1 - (9,9-Dimethyl-9H-xanthen-4,5-diyl)bis(diphenylphosphan) (= Xantphos),
L2 - 10,10'-(Oxybis(2,1-phenylen))bis(10H-phenoxaphosphinin),
L3 - 2,2'-((9,9-Dimethyl-9H-xanthen-4,5-diyl)bis(tert-butylphosphandiyl))dipyridin,
L4 - (Oxybis(2,1-phenylen))bis(tert-butyl-(phenyl)phosphan),
L5 - 4,6-Bis(diphenylphosphanyl)-10H-phenoxazin,
L6 - 1,3-Bis((diphenyl-phosphanyl)methyl)benzen,
L7 - (Oxybis(2,1-phenylen))bis(di-tert-butylphosphan),
L8 - (Oxy-bis(2,1-phenylen))bis(di-o-tolylphosphan),
L9 - Bis(2-(diphenylphosphanyl)-5-methyl--phenyl)methan,
L10 - 1,2-Bis((di-tert-butylphosphanyl)methyl)benzen,
L11 - Di(1-adamantyl)-n-butyl-phosphin,
L12 - 1-(2-(diphenyl-phosphanyl)benzyl)-1H-pyrrol.

Xantphos ist dabei besonders bevorzugt, insbesondere in Kombination mit Pd-Acetylacetonat.

Der Palladiumkatalysator umfasst den Phosphinliganden im Verhältnis Palladium zu Ligand im Bereich von 1:1 bis 1:20, vorzugsweise im Bereich von 1:1 bis 1:10, besonders bevorzugt im Bereich von 1:1 bis 1:2. Das Verhältnis von Palladium zu Halogenwasserstoff liegt im Bereich von 1: 3 bis 1:30. Alle Verhältnisse sind Molverhältnisse.

Wirksame Katalysatormengen im Verfahren liegen vorzugsweise bei 0,01 bis 12 Mol-% Palladium bezogen auf den Allylalkohol, vorzugsweise werden 0,05 mol-% bis 1,5 mol-% Palladium, bezogen auf das Allylalkoholsubstrat eingesetzt.

Für das erfindungsgemäße Verfahren können Lösungsmittel eingesetzt werden. So werden beispielsweise polare inerte organische Lösungsmittel oder/und Wasser verwendet. Zum Beispiel werden dipolar aprotische Lösungsmittel, Ether, aliphatische Ether, Amide, aromatische Verbindungen, Alkohole und Ester, sowie deren Gemische verwendet. Besonders bevorzugt werden aromatische Verbindungen und aliphatische Ether wie Toluol und Diethylether eingesetzt.

Besonders bevorzugt wird im erfindungsgemäßen Verfahren als Halogenwasserstoff Chlorwasserstoff verwendet, vorzugsweise in einem apolaren organischen Lösungsmittel oder Lösungsmittelgemisch. Insbesondere wird die Reaktion in einem Gemisch aus HCl/Diethylether und einem weiteren Lösungsmittel, vorzugsweise Toluol, durchgeführt.

In einer weiteren Ausführungsvariante des Verfahrens kann der Reaktion ein Metallhalogenid, vorzugsweise ein Natrium- oder Lithiumhalogenid, z.B. LiBr oder Lil zugesetzt werden. Dabei beträgt das Verhältnis von Metallhalogenid zum Halogenwasserstoff bevorzugt 1:1 bis 5:1. Besonders bevorzugt liegt das Verhältnis Halogenid zu Halogenwasserstoff bei 1:1 bis 2:1. In einer besonders vorteilhaften Variante wird Chlorwasserstoff zusammen mit LiBr oder Lil eingesetzt.

Überraschenderweise können mit dem erfindungsgemäßen Verfahren die entsprechenden Diester, in der Regel als Isomerengemische, aber auch als bevorzugt n-Verbindungen in guten Ausbeuten hergestellt werden. Insbesondere durch die Zugabe von Lithiumhalogeniden kann das Verhältnis n-/iso-Produkt überraschend positiv zur Bildung der n-Isomeren beeinflusst werden. Das Verfahren stellt somit eine hoch atom- und prozessökonomische Diestersynthese dar. Es werden vier chemische Bindungen auf einmal geknüpft. Überraschend werden Ausbeuten bis zu 75% erreicht.

Die Erfindung wird in nachfolgenden Beispielen näher erläutert.

### Ausführungsbeispiele

### Allgemeine Bemerkungen:

Alle kommerziellen Reagenzien wurden von Alfa Aesar, Aldrich, TCl oder Strem bestellt. Soweit nicht anders gesagt, wurden handelsübliche Reagenzien ohne Reinigung verwendet. Allylalkohol wird im Vakuum vor der Verwendung destilliert. Toluol, DMF, THF, Acetonitril und Methanol werden aus dem Lösungsmittelreinigungssystem PS-MD-7-von "Innovative technology" unter Anwendung von Standard-Schlenk-Techniken verwendet. Analytische Daten der Literatur bekannten Verbindungen waren in Übereinstimmung mit gemeldeten Daten. NMR-Spektren wurden auf Bruker Avance 300 (300 MHz) NMR-Spektrometer aufgezeichnet. Mµltiplets als s (Singµlett), d (Dublett), t (Triplett), dd (Dublett von Dubletts), m (Mµltiplett) und br zugeordnet. s (breites Singµlett). Alle Messungen wurden bei Raumtemperatur durchgeführt, sofern nicht anders angegeben. Elektronenstoß (EI) Massenspektren wurden auf AMD 402-Massenspektrometer (70 eV) aufgenommen. Hochauflösende Massenspektren (HRMS) wurden auf Agilent 6210 aufgezeichnet Time-of-Flight LC / MS (Agilent) mit Elektrospray-Ionisation (ESI). Die Daten werden als Masseeinheiten pro Ladung (m / z) und Intensitäten von Signalen in Klammern angegeben. Die Produkte wurden aus dem Reaktionsgemisch durch Säµlenchromatographie an Kieselgel 60, 0,063-0,2 mm, 70-230 mesh (Merck) getrennt.

### GC Analytik:

GC Analytik wurde mittels einem Agilent GC 7890A Gaschromatographen der Agilent Company mit einer 30 m HP-5 Säµle((Polydimethylsiloxan mit 5% Phenyl-Gruppen, 30 m, 0,32 mm ID, 0,25 & µm Filmdicke). durchgeführt. Temperaturprogramm: 35°C, 10 min; 10°C/min zu 285°C, 5 min; Injektionsvolumen 1 µL mit einem Split von 50:1.

Abkürzungsverzeichnis:
BnOH: Benzylalkohol
CyOH: Cyclohexanol
equiv.: Äquivalente
HCl: Chlorwasserstoff
LSM: Lösungsmittel
THF: Tetrahydrofuran
T: Temperatur
p: Druck
Xantphos: 4,5-Bis(diphenylphosphanyl)-9,9-dimethylxanthen

### Beispiel 1

Verwendung verschiedener Allylalkohole (Tabelle 1):

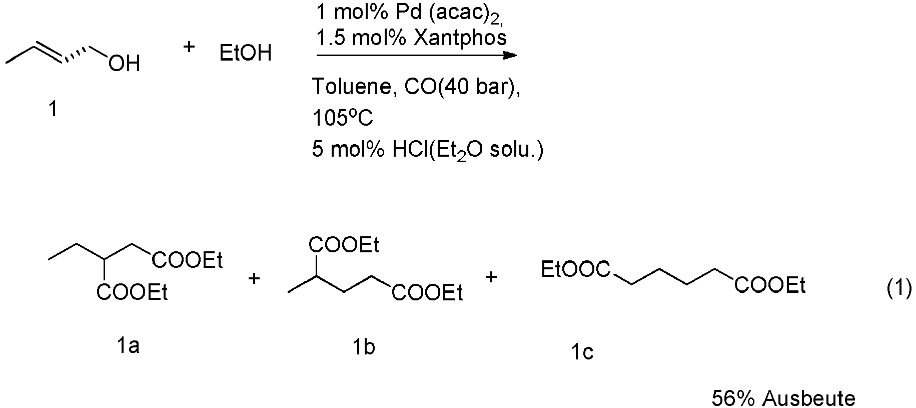

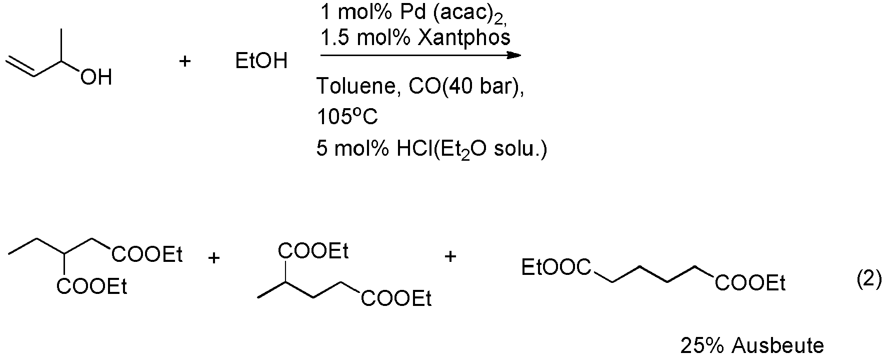

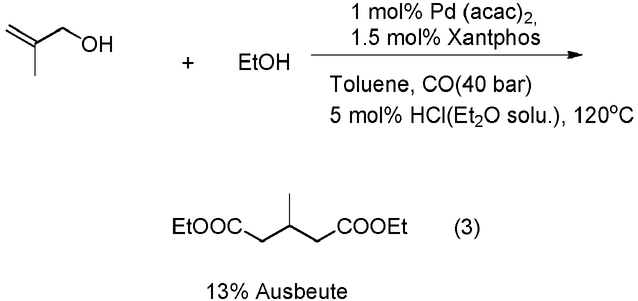

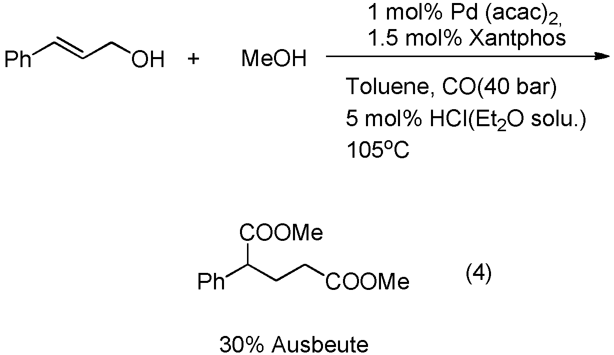

**Tabelle 1**

| Eintrag | Pd (mol%) | Ligand (mol%) | LSM | Säure (mol%) | T[°C] | CO p(bar) | Ausbeute an isomeren Diestern[%] |
|---|---|---|---|---|---|---|---|
| 1 | Pd(acac)₂(1 ) | Xantphos(1.5) | Toluol | HCl (5) | 105 | 40 | 56 |
| 2 | Pd(acac)₂(1 ) | Xantphos(1.5) | Toluol | HCl (5) | 105 | 40 | 25 |
| 3 | Pd(acac)₂(1 ) | Xantphos(1.5) | Toluol | HCl (5) | 105 | 40 | 13 |
| 4 | Pd(acac)₂(1 ) | Xantphos(1.5) | Toluol | HCl (5) | 105 | 40 | 30 |

### Beispiel 1.1

### Tabelle 1, Eintrag 1

Ein 4 ml Glasvial wird mit [Pd(acac)₂] (3.77 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Crotylalkohol (87 µl, 1 mmol), Ethanol (175 µl, 3 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 1.2

### Tabelle 1, Eintrag 2

Ein 4 ml Glasvial wird mit [Pd(acac)₂] (3.77 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, But-3-en-2-ol (87 µl, 1 mmol), Ethanol (175 µl, 3 mmol) und 1 M Salzsäure Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 1.3

### Tabelle 1, Eintrag 3

Ein 4 ml Glasvial wird mit [Pd(acac)₂] (3.77 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, 2-Methylprop-2-en-1-ol (87 µl, 1 mmol), Ethanol (175 µl, 3 mmol) und 1 M Salzsäure Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt

### Beispiel 1.4

### Tabelle 1, Eintrag 4

Ein 4 ml Glasvial wird mit [Pd(acac)₂] (3.77 mg, 1 mol%), Xantphos (8.8 mg, 1.5 mol%), Cinnamylalkohol (134 mg, 1 mmol) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, 3-Phenylprop-2-en-1-ol (87 µl, 1 mmol), Methanol (123 µl, 3 mmol) und 1 M Salzsäure Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt

### Beipiele 2

Variation der Alkohole(Tabelle 2):

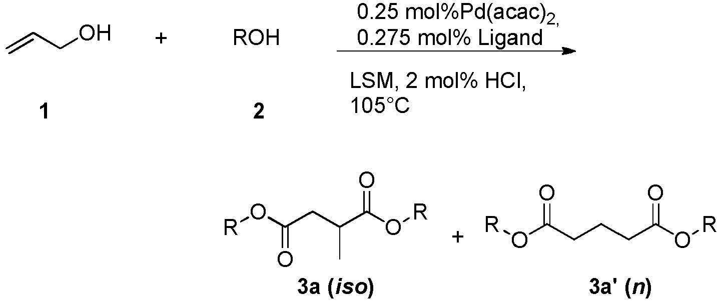

**Tabelle 2**

| Eintrag | ROH | Ausbeute(n:iso) |
|---|---|---|
| 1 | MeOH | 38(58:42) |
| 2 | EtOH | 80(58:42) |
| 3 | BuOH | 77(55:45) |
| 4 | i-PrOH | 91(60:40) |
| 5 | BnOH | 80 (62:38) |

### Beispiel 2.1

### Tabelle 2, Eintrag 1

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Methanol (123 µl, 3 mmol), Allylalkohol (68 µl, 1 mmol), und 1 M Salzsäure Diethyletherlösung (20 µl, 2 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 2.2

### Tabelle 2, Eintrag 2

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Ethanol (175 µl, 3 mmol), Allylalkohol (68 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 2.3

### Tabelle 2, Eintrag 3

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 2.4

### Tabelle 2, Eintrag 4

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Isopropanol (230 µl, 3 mmol), Allylalkohol (68 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 2.5

### Tabelle 2, Eintrag 5

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, Benzylalkohol (310 µl, 3 mmol), Allylalkohol (68 µl, 1 mmol), und 1 M Salzsäure Diethyletherlösung (20 µl, 2 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3

Variation des Palladiumprecursors (Tabelle 3)

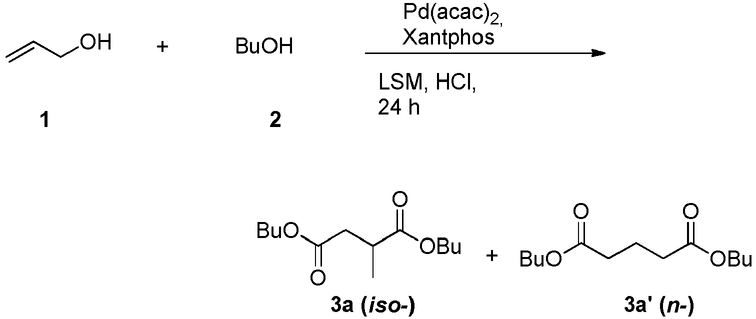

**Tabelle 3**

| Eintrag | [Pd](mol%) | L(mol%) | LSM | Säure(mol%) | T[°C] | p[bar] | Ausbeute[%] (n:iso) |
|---|---|---|---|---|---|---|---|
| 1 | Pd(acac)₂(1 ) | Xantphos(1.5) | Toluol | HCl (5) | 105 | 40 | 69 (54:46) |
| 2 | Pd(OAc)₂(1) | Xantphos(1.5) | Toluol | HCl (5) | 105 | 40 | 64(53:47) |
| 3 | PdCl₂(1 ) | Xantphos(1.5) | Toluol | HCl (5) | 105 | 40 | 61(53:47) |
| 4 | PdBr₂(1 ) | Xantphos(1.5) | Toluol | HCl (5) | 105 | 40 | 42(63:37) |
| 5 | Pdl₂(1 ) | Xantphos(1.5) | Toluol | HCl (5) | 105 | 40 | 24(78:22) |
| 6 | Pd(COD)Cl₂(1 ) | Xantphos(1.5) | Toluol | HCl (5) | 105 | 40 | 62(53:47) |
| 7 | Pd₂(dba)₃(0.5) | Xantphos(1.5) | Toluol | HCl (5) | 105 | 40 | 70(57:43) |
| 8 | Pd(TFA)₂ | Xantphos(1.5) | Toluol | HCl (5) | 105 | 40 | 72(56:44) |
| 9 | PdO(1 ) | Xantphos(1.5) | Toluol | HCl (5) | 105 | 40 | 64(54:46) |
| 10 | Pd(NO₃)₂·2H₂O | Xantphos(1.5) | Toluol | HCl (5) | 105 | 40 | 46(54:46) |
| 11 | (NH₄)₂PdCl₆ | Xantphos(1.5) | Toluol | HCl (5) | 105 | 40 | 49(52:48) |

### Beispiel 3.1

### Tabelle 3, Eintrag 1

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.2

### Tabelle 3, Eintrag 2

Ein 4 ml Glasvial wird mit [Pd(OAc)₂] (2.25 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.3

### Tabelle 3, Eintrag 3

Ein 4 ml Glasvial wird mit [PdCl₂] (1.8 mg, 1 mol%), Xantphos (8.8 mg, 1.5 mol% und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.4

### Tabelle 3, Eintrag 4

Ein 4 ml Glasvial wird mit [PdBr₂] (2.7 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.5

### Tabelle 3, Eintrag 5

Ein 4 ml Glasvial wird mit [Pdl₂] (3,6 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.6

### Tabelle 3, Eintrag 6

Ein 4 ml Glasvial wird mit [Pd(COD)Cl₂] (2.9 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.7

### Tabelle 3, Eintrag 7

Ein 4 ml Glasvial wird mit [Pd₂(dba)₃] (4.6 mg, 0.5 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.8

### Tabelle 3, Eintrag 8

Ein 4 ml Glasvial wird mit [Pd(TFA)₂] (3.4 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.9

### Tabelle 3, Eintrag 9

Ein 4 ml Glasvial wird mit PdO (1.21 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.10

### Tabelle 3, Eintrag 10

Ein 4 ml Glasvial wird mit Pd(NO₃)₂·2H₂O (2.7 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 3.11

### Tabelle 3, Eintrag 11

Ein 4 ml Glasvial wird mit (NH₄)₂PdCl₆ (3.5 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar Co aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt

### Beispiel 4

Variation der Lösungsmittel (Tabelle 4):

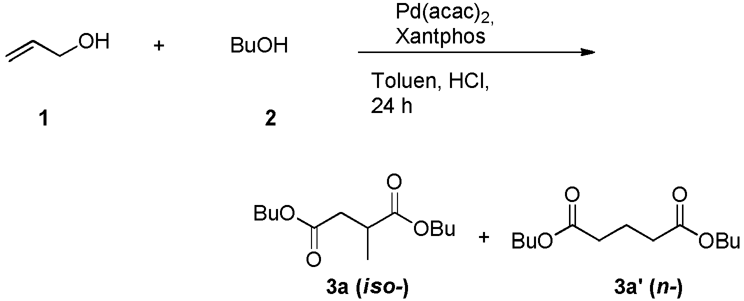

**Tabelle 4**

| Eintrag | Pd (mol%) | Ligand (mol%) | LSM | Säure(mol%) | T(°C) | p(bar) | Ausbeute 3a+3a' (***n-*/*****iso**-*) |
|---|---|---|---|---|---|---|---|
| 1 | Pd(acac)₂(1 ) | Xantphos(1.5) | Toluol | HCl (5) | 105 | 40 | 70 (58:42) |
| 2 | Pd(acac)₂(1 ) | Xantphos(1.5) | Dioxan | HCl (5) | 105 | 40 | 54 (56:44) |
| 3 | Pd(acac)₂(1 ) | Xantphos(1.5) | Aceton | HCl (5) | 105 | 40 | 58(63:37) |
| 4 | Pd(acac)₂(1 ) | Xantphos(1.5) | Butanol | HCl (5) | 105 | 40 | 8 (76:24) |
| 5 | Pd(acac)₂(1 ) | Xantphos(1.5) | THF | HCl (5) | 105 | 40 | 41 (60:40) |
| 6 | Pd(acac)₂(1 ) | Xantphos(1.5) | MeCN | HCl (5) | 105 | 40 | 55 (72:28) |
| 7 | Pd(acac)₂(1 ) | Xantphos(1.5) | Anisol | HCl (5) | 105 | 40 | 58 (56:44) |

### Beispiel 4.1

### Tabelle 4, Eintrag 1

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Toluol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 4.2

### Tabelle 4, Eintrag 2

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Dioxan (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 4.3

### Tabelle 4, Eintrag 3

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Aceton (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 4.4

### Tabelle 4, Eintrag 4

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun n-Butanol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 4.5

### Tabelle 4, Eintrag 5

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun THF (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 4.6

### Tabelle 4, Eintrag 6

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Acetonitril (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 4.7

### Tabelle 4, Eintrag 7

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun Anisol (2 mL), Allylalkohol (68 µl, 1 mmol), n-Butanol (182 µl, 2 mmol) und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5

Variation Druck und Temperatur, Säuremenge, Ligandverhältnis, (Tabelle 5):

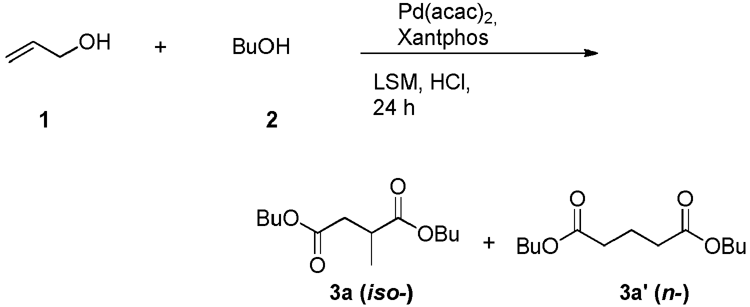

**Tabelle 5**

| Eintrag | Pd (mol%) | Ligand (mol%) | Lösungsmittel | Säure (mol%) | T[°C] | CO p(bar) | Ausbeute(***n-*/*iso*-**) |
|---|---|---|---|---|---|---|---|
| 1 | Pd(acac)₂(1 ) | Xantphos(1.5) | Toluol | HCl (5) | 89 | 40 | 70 (58:42) |
| 2 | Pd(acac)₂(1 ) | Xantphos(1.5) | Toluol | HCl (5) | 80 | 40 | 54 (56:44) |
| 3 | Pd(acac)₂(0.1 ) | Xantphos(0.15) | Toluol | HCl (2) | 140 | 40 | 67(53) |
| 4 | Pd(acac)₂(0.25) | Xantphos(0.275) | Toluol | HCl (2) | 105 | 10 | 15(62) |
| 5 | Pd(acac)₂(0.25) | Xantphos(0.275) | Toluol | HCl (2) | 105 | 20 | 59(60) |
| 6 | Pd(acac)₂(0.25) | Xantphos(0.275) | Toluol | HCl (2) | 105 | 40 | 73(58) |
| 7 | Pd(acac)₂(0.25) | Xantphos(0.275) | Toluol | HCl (2) | 105 | 60 | 66(59) |
| 8 | Pd(acac)₂(0.25) | Xantphos(0.275) | Toluol | HCl (2) | 120 | 40 | 74(56) |
| 9 | Pd(acac)₂(0.25) | Xantphos(0.275) | Toluol | HCl (2) | 140 | 40 | 70(55) |

### Beispiel 5.1

### Tabelle 5, Eintrag 1

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 89 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.2

### Tabelle 5, Eintrag 2

Ein 4 ml Glasvial wird mit [Pd(acac)₂] (3.04 mg, 1 mol%), Xantphos (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 80 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.3

### Tabelle 5, Eintrag 3

Ein 4 ml Glasvial wird mit [Pd(acac)₂] (0.30 mg, 0.1 mol%), Xantphos (0.89 mg, 0.15 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 140°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.4

### Tabelle 5, Eintrag 4

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 10 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.5

### Tabelle 5, Eintrag 5

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 20 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.6

### Tabelle 5, Eintrag 6

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.7

### Tabelle 5, Eintrag 7

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 60 bar CO aufgepresst. Die Reaktion wird 24 Stunden bei 105°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.8

### Tabelle 5, Eintrag 8

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 120°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 5.9

### Tabelle 5, Eintrag 9

Ein 4 ml Glasvial wird mit [Pd(acac)₂] (0.77 mg, 0.25 mol%), Xantphos (1.62 mg, 0.275 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (273 µl, 3 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 140°C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6

Variation der Säure (Tabelle 6)

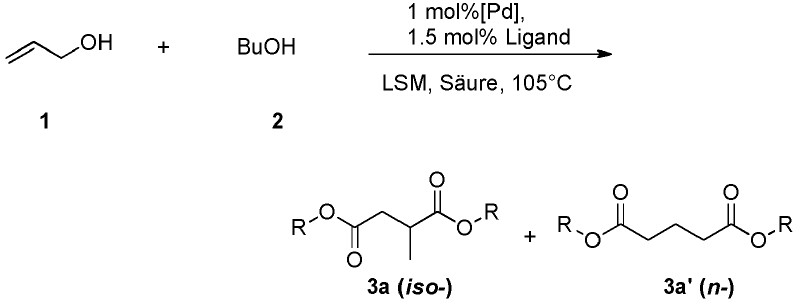

**Tabelle 6**

| Eintrag | Pd (mol%) | Ligand (mol%) | LSM | Säure (mol%) | T(°C) | p(bar) | Ausbeute(***n-*/*iso*-**) |
|---|---|---|---|---|---|---|---|
| 1 | Pd(acac)₂(1 ) | Xantphos(1.5) | Toluol | HCl (5) | 105 | 40 | 77 (54:46) |
| 2 | Pd(acac)₂(1 ) | Xantphos(1.5) | Toluol | TsOH H₂O (5) | 105 | 40 | 0 |
| 3 | Pd(acac)₂(1 ) | Xantphos(1.5) | Toluol | TFA (5) | 105 | 40 | 0 |
| 4 | Pd(acac)₂(1 ) | Xantphos(1.5) | Toluol | MSA (5) | 105 | 40 | 0 |
| 5 | Pd(acac)₂(1 ) | Xantphos(1.5) | Toluol | KCl (5) | 105 | 40 | 0 |
| 6 | Pd(acac)₂(1 ) | Xantphos(1.5) | Toluol | LiCI (5) | 105 | 40 | 0 |
| 7 | Pd(acac)₂(1 ) | Xantphos(1.5) | THF | HBr(5) wässrige Lösung | 105 | 40 | 10(73:27) |
| 8 | Pd(acac)₂(1 ) | Xantphos(1.5) | THF | HCl (5) wässrige Lösung | 105 | 40 | 15(62:38) |
| 9 | Pd(acac)₂(1 ) | Xantphos(1.5) | Toluol | HCl (5) Etherlösung+ LiBr(5) | 105 | 40 | 32(67:33) |
| 10 | Pd(acac)₂(1 ) | Xantphos(1.5) | Toluol | HCl (5) Etherlösung+ LiBr(10) | 105 | 40 | 25 (65:33) |
| 11 | Pd(acac)₂(1 ) | Xantphos(1.5) | Toluol | HCl (5) Etherlösung+ Lil(5) | 105 | 40 | 10(84:16) |
| 12 | Pd(acac)₂(1 ) | Xantphos(1.5) | Toluol | HCl(5) Etherlösung+ Lil(10) | 105 | 40 | 10(82:18) |

### Beispiel 6.1

### Tabelle 6, Eintrag 1

Ein 4 ml Glasvial wird mit [Pd(acac)₂] (3.04 mg, 1 mol%), L1 (Xantphos), (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol), und 1 M Salzsäure Diethyletherlösung (50 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.2

### Tabelle 6, Eintrag 2

Ein 4 ml Glasvial wird mit [Pd(acac)₂] (3.07 mg, 1 mol%), L1 (Xantphos), (8.8 mg, 1.5 mol%), TsOH·H₂O (9,5 mg, 5 mol%), und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.3

### Tabelle 6, Eintrag 3

Ein 4 ml Glasvial wird mit [Pd(acac)₂] (3.07 mg, 1 mol%), L1 (Xantphos), (8.8 mg, 1.5 mol%), und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und Trifluoressigsäure (TFA) (3,8 µl, 5 mol%) mittels Spritzen injiziert. Dieses Vial wird nun in einer Metallplatte platziert welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.4

### Tabelle 6, Eintrag 4

Ein 4 ml Glasvial wird mit [Pd(acac)₂] (3.07 mg, 1 mol%), L1 (Xantphos), (8.8 mg, 1.5 mol%), und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und Methansµlfonsäure (MSA), (3,2 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.5

### Tabelle 6, Eintrag 5

Ein 4 ml Glasvial wird mit [Pd(acac)₂] (3.07 mg, 1 mol%), L1 (Xantphos), (8.8 mg, 1.5 mol%), Kaliumchlorid (3.7 mg, 5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.6

### Tabelle 6, Eintrag 6

Ein 4 ml Glasvial wird mit [Pd(acac)₂] (3.07 mg, 1 mol%), L1 (Xantphos), (8.8 mg, 1.5 mol%), Lithiumchlorid (2,1 mg, 5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.7

### Tabelle 6, Eintrag 7

Ein 4 ml Glasvial wird mit [Pd(acac)₂] (3.07 mg, 1 mol%), L1 (Xantphos), (8.8 mg, 1.5 mol%), und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml THF, n-Butanol (182 µl, 2 mmol), Allylalkohol (68 µl, 1 mmol) und 1M HBr wässrige Lösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.8

### Tabelle 6, Eintrag 8

Ein 4 ml Glasvial wird mit [Pd(acac)₂] (3.07 mg, 1 mol%), L1 (Xantphos), (8.8 mg, 1.5 mol%), und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml THF, n-Butanol (182 µl, 2 mmol), Allylalkohol (68 µl, 1 mmol) und 1M HCl wässrige Lösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.9

### Tabelle 6,Eintrag 9

Ein 4 ml Glasvial wird mit [Pd(acac)₂] (3.07 mg, 1 mol%), L1 (Xantphos), (8.8 mg, 1.5 mol%), LiBr (4.5 mg, 5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.10

### Tabelle 6,Eintrag 10

Ein 4 ml Glasvial wird mit [Pd(acac)₂] (3.07 mg, 1 mol%), L1 (Xantphos), (8.8 mg, 1.5 mol%), LiBr (9 mg, 10 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.11

### Tabelle 6,Eintrag 11

Ein 4 ml Glasvial wird mit [Pd(acac)₂] (3.07 mg, 1 mol%), L1 (Xantphos), (8.8 mg, 1.5 mol%), Lil (6.6 mg, 5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 6.12

### Tabelle 6,Eintrag 12

Ein 4 ml Glasvial wird mit [Pd(acac)₂] (3.07 mg, 1 mol%), L1 (Xantphos), (8.8 mg, 1.5 mol%), Lil (13.3 mg, 10 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7

Ligandvariation (Tabelle 7):

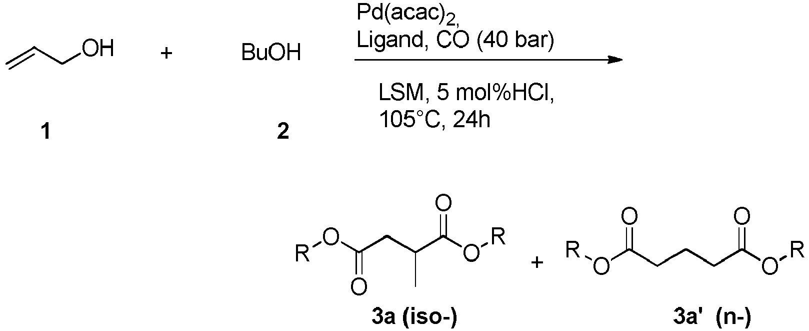

**Tabelle 7**

| Eintrag | | Ligand | Ausbeute% (n : iso) |
|---|---|---|---|
| 1 | L1 | 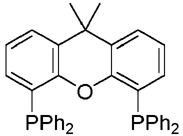 | 71% (n: iso = 54:49) |
| 2 | L2 | 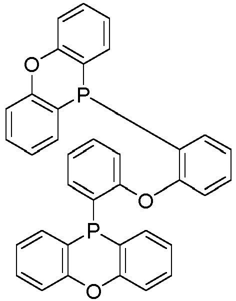 | 28% n: iso = 34:66 |
| 3 | L3 | 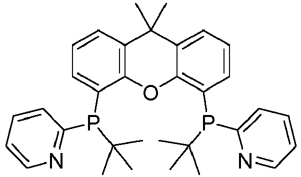 | 41% n: iso = 49:51 |
| 4 | L4 | 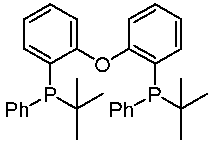 | 28% n: iso = 27:73) |
| 5 | L5 | 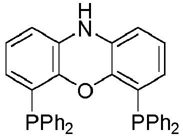 | 56% n: iso = 47:53 |
| 6 | L6 | 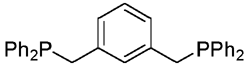 | 24% n: iso = 34:66 |
| 7 | L7 | 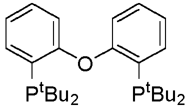 | 13% n: iso =25:75 |
| 8 | L8 | 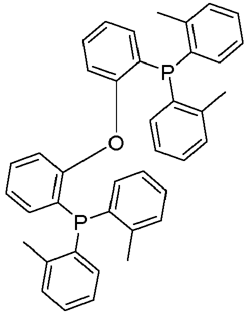 | R = 2-Me-Ph 29% n: iso = 15:85 |
| 9 | L9 | 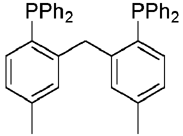 | 53% n: iso = 85:15 |
| 10 | L10 | 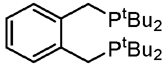 | 15% n: iso = 64:36 |
| 11 | L11 | BuPAd₂ | 31% n: iso = 25:75 |
| 12 | L12 | 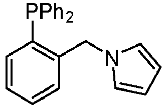 | 15% n: iso = 21:79 |

### Beispiel 7.1

### Tabelle 7, Eintrag 1

Ein 4 ml Glasvial wird mit [Pd(acac)₂] (3.07 mg, 1 mol%), L1 (Xantphos), (8.8 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.2

### Tabelle 7, Eintrag 2

Ein 4 ml Glasvial wird mit [Pd(acac)₂] (3.07 mg, 1 mol%), L2 (10,10'-(Oxybis(2,1-phenylen))bis(10H-phenoxaphosphinin)), (8.5 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.3

### Tabelle 7, Eintrag 3

Ein 4 ml Glasvial wird mit [Pd(acac)₂] (3.07 mg, 1 mol%), L3 (2,2'-((9,9-Dimethyl-9H-xanthen-4,5-diyl)bis(tert-butylphosphanediyl))dipyridin), (8.1 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.4

### Tabelle 7, Eintrag 4

Ein 4 ml Glasvial wird mit [Pd(acac)₂] (3.07 mg, 1 mol%), L4 ((Oxybis(2,1-phenylen))bis(tert-butyl(phenyl)phosphan)), (7.5 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.5

### Tabelle 7, Eintrag 5

Ein 4 ml Glasvial wird mit [Pd(acac)₂] (3.07 mg, 1 mol%), L5 (4,6-Bis(diphenylphosphanyl)-10H-phenoxazin), (8.3 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.6

### Tabelle 7, Eintrag 6

Ein 4 ml Glasvial wird mit [Pd(acac)₂] (3.07 mg, 1 mol%), L6 (1,3-Bis((diphenylphosphanyl)methyl)benzen), (7.1 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.7

### Tabelle 7, Eintrag 7

Ein 4 ml Glasvial wird mit [Pd(acac)₂] (3.07 mg, 1 mol%), L7 ((Oxybis(2,1-phenylen))bis(di-tert-butylphosphan)), (6.9 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.8

### Tabelle 7, Eintrag 8

Ein 4 ml Glasvial wird mit [Pd(acac)₂] (3.07 mg, 1 mol%), L8((Oxybis(2,1-phenylen))bis(di-o-tolylphosphan)), (8.9 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.9

### Tabelle 7, Eintrag 9

Ein 4 ml Glasvial wird mit [Pd(acac)₂] (3.07 mg, 1 mol%), L9 (Bis(2-(diphenylphosphanyl)-5-methylphenyl)methan), (8.5 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

### Beispiel 7.10

### Tabelle 7, Eintrag 10

Ein 4 ml Glasvial wird mit [Pd(acac)₂] (3.07 mg, 1 mol%), L10 (1,2-Bis((di-tert-butylphosphanyl)methyl)benzen), (5.91 mg, 1.5 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt

### Beispiel 7.11

### Tabelle 7, Eintrag 11

Ein 4 ml Glasvial wird mit [Pd(acac)₂] (3.07 mg, 1 mol%), L11 (Di-1-adamantylbutylphosphan), (11 mg, 3 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt

### Beispiel 7.12

### Tabelle 7, Eintrag 12

Ein 4 ml Glasvial wird mit [Pd(acac)₂] (3.07 mg, 1 mol%), L12 (1-(2-(diphenylphosphanyl)benzyl)-1H-pyrrol), (10.2 mg, 3 mol%) und einem Magnetrührer versehen. Dieses Vial wird mit einer Kappe aus Phenoplaste verschlossen, welches mit einem Septum aus teflonbeschichtetem Styrol-Butadiengummi besteht. Durch dieses Septum wird eine Kanüle gestochen und durch diese Kanüle wird durch dreimaliges Vakuumanlegen und Argonspülen die Atmosphäre in dem Vial durch eine Argonatmosphäre ersetzt. Durch diese Kanüle werden nun 2 ml Toluol, n-Butanol (182 µl, 2 mmol), Allylalkohol (68 µl, 1 mmol) und 1 M HCl-Diethyletherlösung (50 µl, 5 mol%) mittels Spritzen injiziert. Dieses Vial wird nun in einer Metallplatte platziert, welche dann in einen 300 ml Stahlautoklav der Firma Parr Instruments unter Argonatmosphäre überführt wird. Nachdem der Autoklav dreimal mit CO gespült wurde, werden bei Raumtemperatur 40 bar CO aufgepresst. Die Reaktion wird 24 Stunden bei 105 °C unter Magnetrührung durchgeführt. Nachdem Ende der Reaktion wird der Autoklav heruntergekühlt und der Druck langsam abgelassen. Der Autoklav wird dreimal mit Stickstoff gespült. Anschließend werden Isooctan (100 µl) als interner Standard zugesetzt. Die Ausbeute und Selektivität wird mittels GC Analyse bestimmt.

## Patentansprüche

1. Verfahren zur doppelten Carbonylierung von Allylalkoholen zu Diestern,
**dadurch gekennzeichnet, dass** ein linearer oder verzweigter Allylalkohol mit einem linearen oder verzweigtem Alkanol unter Zuführung von CO und in Gegenwart eines katalytischen Systems aus einem Palladiumkomplex und mindestens einem organischen Phosphorliganden sowie in Anwesenheit eines Halogenwasserstoffs ausgewählt aus HCl, HBr oder HJ zur Reaktion gebracht wird.

2. Verfahren nach Anspruch 1,
**dadurch gekennzeichnet, dass** die Allylalkohole Verbindungen der allgemeinen Formel (1) darstellen: 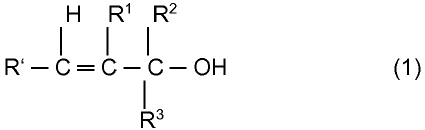
wobei R¹, R² und R³ unabhängig voneinander Wasserstoff oder einen C₁ bis C₁₀ Alkylrest bedeuten und
R' für Wasserstoff, oder einen gesättigten verzweigten oder unverzweigten aliphatischen, cycloaliphatischen oder cycloaliphatisch-aliphatischen Kohlenwasserstoffrest mit bis zu 12 C-Atomen, in dem C-C-Bindungen durch Sauerstoff oder die -O-CO-Gruppe unterbrochen sein können, oder einen Phenylrest steht, wobei der Phenylrest wie folgt substituiert sein kann: C₁- bis C₁₀-Alkyl- oder C₁-bis C₁₀-Alkoxy-Gruppen.

3. Verfahren nach Anspruch 1 oder 2,
**dadurch gekennzeichnet, dass** die Alkanole Verbindungen der allgemeinen Formel ROH darstellen, wobei R eine C₁- bis C₂₀-Alkyl-, eine C₁- bis C₂₀-Cycloalkyl- oder eine C₇- bis C₁₁-Aralkylgruppe ist.

4. Verfahren nach einem der Ansprüche 1 bis 3,
**dadurch gekennzeichnet, dass** die Reaktion in flüssiger Phase bei einer Temperatur von 70 bis 250 °C durchgeführt wird.

5. Verfahren nach einem der Ansprüche 1 bis 4,
**dadurch gekennzeichnet, dass** Reaktion unter einem Druck von 2 bis 100 bar durchgeführt wird.

6. Verfahren nach einem der Ansprüche 1 bis 5,
**dadurch gekennzeichnet, dass** der Palladiumkomplex in-situ ausgehend von einem Vorkomplex gebildet wird, wobei als Palladiumquelle Palladium-enthaltende Salze und Komplexe als Vorstufe verwendet werden.

7. Verfahren nach Anspruch 6,
**dadurch gekennzeichnet, dass** der Palladiumkomplex ausgewählt ist aus der Gruppe enthaltend Pd-Acetat, Pd-Acetylacetonat, Pd-Halogenide, Pd-Halogen-1,5-cyclooctadiene, Pd-Nitrate, Pd-Oxid und Diammoniumhexachloropalladat.

8. Verfahren nach einem der Ansprüche 1 bis 7,
**dadurch gekennzeichnet, dass** die Phosphinliganden eine mono- oder bidentate Struktur aufweisen, vorzugsweise ausgewählt sind aus der Gruppe umfassend:
L1 - (9,9-Dimethyl-9H-xanthen-4,5-diyl)bis(diphenylphosphan),
L2 - 10,10'-(Oxybis(2,1-phenylen))bis(10H-phenoxaphosphinin),
L3 - 2,2'-((9,9-Dimethyl-9H-xanthen-4,5-diyl)bis(tert-butylphosphandiyl))dipyridin,
L4 - (Oxybis(2,1-phenylen))bis(tert-butyl-(phenyl)phosphan),
L5 - 4,6-Bis(diphenylphosphanyl)-10H-phenoxazin,
L6 - 1,3-Bis((diphenyl-phosphanyl)methyl)benzen,
L7 - (Oxybis(2,1-phenylen))bis(di-tert-butylphosphan),
L8 - (Oxy-bis(2,1-phenylen))bis(di-o-tolylphosphan),
L9 - Bis(2-(diphenylphosphanyl)-5-methyl--phenyl)methan,
L10 - 1,2-Bis((di-tert-butylphosphanyl)methyl)benzen,
L11 - Di(1-adamantyl)-n-butyl-phosphin,
L12 - 1-(2-(diphenyl-phosphanyl)benzyl)-1H-pyrrol.

9. Verfahren nach einem der Ansprüche 1 bis 8,
**dadurch gekennzeichnet, dass** als Halogenwasserstoff Chlorwasserstoff verwendet wird.

10. Verfahren nach einem der Ansprüche 1 bis 9,
**dadurch gekennzeichnet, dass** das Verhältnis von Palladium zu Halogenwasserstoff im Bereich von 1: 3 bis 1:30 liegt.

11. Verfahren nach einem der Ansprüche 1 bis 10,
**dadurch gekennzeichnet, dass** der Reaktion ein Metallhalogenid zugesetzt wird, wobei das Verhältnis Halogenid zu Halogenwasserstoff im Bereich von 1:1 bis 5:1 liegt.

## Claims

1. Process for doubly carbonylating allyl alcohols to diesters,
**characterized in that** a linear or branched allyl alcohol is reacted with a linear or branched alkanol with supply of CO and in the presence of a catalytic system composed of a palladium complex and at least one organic phosphorus ligand and in the presence of a hydrogen halide selected from HCl, HBr and HI.

2. Process according to Claim 1,
**characterized in that** the allyl alcohols are compounds of the general formula (1): 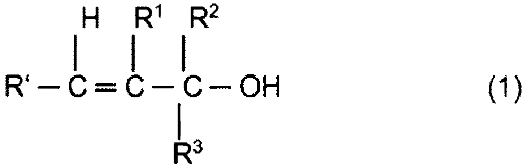
where R¹, R² and R³ are independently hydrogen or a C₁ to C₁₀ alkyl radical and
R' is hydrogen, or a saturated branched or unbranched, aliphatic, cycloaliphatic or cycloaliphatic-aliphatic hydrocarbyl radical having up to 12 carbon atoms, in which C-C bonds may be interrupted by oxygen or the -O-CO- group, or a phenyl radical, where the phenyl radical may be substituted as follows: C₁- to C₁₀-alkyl- or C₁-to C₁₀-alkoxy groups.

3. Process according to Claim 1 or 2,
**characterized in that** the alkanols are compounds of the general formula ROH where R is a C₁- to C₂₀-alkyl, a C₁- to C₂₀-cycloalkyl or a C₇- to C₁₁-aralkyl group.

4. Process according to any of Claims 1 to 3,
**characterized in that** the reaction is conducted in the liquid phase at a temperature of 70 to 250°C.

5. Process according to any of Claims 1 to 4,
**characterized in that** reaction is conducted under a pressure of 2 to 100 bar.

6. Process according to any of Claims 1 to 5,
**characterized in that** the palladium complex is formed in situ proceeding from a pre-complex, using, as palladium source, palladium-containing salts and complexes as precursor.

7. Process according to Claim 6,
**characterized in that** the palladium complex is selected from the group comprising Pd acetate, Pd acetylacetonate, Pd halides, Pd-halogen-1,5-cyclooctadienes, Pd nitrates, Pd oxide and diammonium hexachloropalladate.

8. Process according to any of Claims 1 to 7,
**characterized in that** the phosphine ligands have a mono- or bidentate structure, preferably selected from the group comprising
L1 - (9,9-dimethyl-9H-xanthene-4,5-diyl)bis(diphenylphosphine),
L2 - 10,10'-(oxybis(2,1-phenylene))bis(10H-phenoxaphosphinine),
L3 - 2,2'-((9,9-dimethyl-9H-xanthene-4,5-diyl)bis(tert-butylphosphinediyl))dipyridine,
L4 - (oxybis(2,1-phenylene))bis(tert-butyl(phenyl)phosphine),
L5 - 4,6-bis(diphenylphosphinyl)-10H-phenoxazine,
L6 - 1,3-bis((diphenylphosphinyl)methyl)benzene,
L7 - (oxybis(2,1-phenylene))bis(di-tert-butylphosphine),
L8 - (oxybis(2,1-phenylene))bis(di-o-tolylphosphine),
L9 - bis(2-(diphenylphosphinyl)-5-methylphenyl)methane,
L10 - 1,2-bis((di-tert-butylphosphinyl)methyl)benzene,
L11 - di(1-adamantyl)-n-butylphosphine,
L12 - 1-(2-(diphenylphosphinyl)benzyl)-1H-pyrrole.

9. Process according to any of Claims 1 to 8, **characterized in that** the hydrogen halide used is hydrogen chloride.

10. Process according to any of Claims 1 to 9, **characterized in that** the ratio of palladium to hydrogen halide is in the range from 1:3 to 1:30.

11. Process according to any of Claims 1 to 10, **characterized in that** a metal halide is added to the reaction, where the ratio of halide to hydrogen halide is in the range from 1:1 to 5:1.

## Revendications

1. Procédé de carbonylation double d'alcools allyliques en diesters, **caractérisé en ce qu'**un alcool allylique linéaire ou ramifié est mis en réaction avec un alcanol linéaire ou ramifié avec introduction de CO et en présence d'un système catalytique constitué par un complexe de palladium et au moins un ligand phosphore organique, et en présence d'un halogénure d'hydrogène choisi parmi HCl, HBr ou HI.

2. Procédé selon la revendication 1, **caractérisé en ce que** les alcools allyliques sont des composés de formule générale (1) : 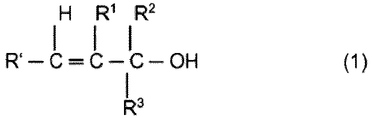
dans laquelle R¹, R² et R³ signifient indépendamment les uns des autres l'hydrogène ou un radical alkyle en C₁ à C₁₀, et
R' représente l'hydrogène ou un radical hydrocarboné saturé, ramifié ou non ramifié, aliphatique, cycloaliphatique ou cycloaliphatique-aliphatique, contenant jusqu'à 12 atomes C, dans lequel les liaisons C-C peuvent être interrompues par de l'oxygène ou le groupe -O-CO-, ou un radical phényle, le radical phényle pouvant être substitué de la manière suivante : groupes alkyle en C₁ à C₁₀ ou alcoxy en C₁ à C₁₀.

3. Procédé selon la revendication 1 ou 2, **caractérisé en ce que** les alcanols sont des composés de formule générale ROH, dans laquelle R est un groupe alkyle en C₁ à C₂₀, cycloalkyle en C₁ à C₂₀ ou aralkyle en C₇ à C₁₁.

4. Procédé selon l'une quelconque des revendications 1 à 3, **caractérisé en ce que** la réaction est réalisée en phase liquide à une température de 70 à 250 °C.

5. Procédé selon l'une quelconque des revendications 1 à 4, **caractérisé en ce que** la réaction est réalisée sous une pression de 2 à 100 bar.

6. Procédé selon l'une quelconque des revendications 1 à 5, **caractérisé en ce que** le complexe de palladium est formé in situ à partir d'un pré-complexe, des sels et des complexes contenant du palladium étant utilisés en tant que source de palladium en tant que précurseurs.

7. Procédé selon la revendication 6, **caractérisé en ce que** le complexe de palladium est choisi dans le groupe contenant l'acétate de Pd, l'acétylacétonate de Pd, les halogénures de Pd, les halogéno-1,5-cyclooctadiènes de Pd, les nitrates de Pd, l'oxyde de Pd et l'hexachloropalladate de diammonium.

8. Procédé selon l'une quelconque des revendications 1 à 7, **caractérisé en ce que** les ligands phosphine présentent une structure mono- ou bidentate, sont de préférence choisis dans le groupe comprenant
L1 - le (9,9-diméthyl-9H-xanthène-4,5-diyl)bis(diphénylphosphane),
L2 - la 10,10'-(oxybis(2,1-phénylène))bis(10H-phénoxaphosphinine),
L3 - la 2,2'-((9,9-diméthyl-9H-xanthéne-4,5-diyl)bis(tert-butylphosphandiyl))dipyridine,
L4 - l'(oxybis(2,1-phénylène))bis(tert-butyl-(phényl)phosphane),
L5 - la 4,6-bis(diphénylphosphanyl)-10H-phénoxazine,
L6 - le 1,3-bis((diphényl-phosphanyl)méthyl)benzène,
L7 - l'(oxybis(2,1-phénylène))bis(di-tert-butylphosphane),
L8 - l'(oxy-bis(2,1-phénylène))bis(di-o-tolylphosphane),
L9 - le bis(2-(diphénylphosphanyl)-5-méthyl-phényl)méthane,
L10 - le 1,2-bis((di-tert-butylphosphanyl)méthyl)benzène,
L11 - la di(1-adamantyl)-n-butyl-phosphine,
L12 - le 1-(2-(diphényl-phosphanyl)benzyl)-1H-pyrrol.

9. Procédé selon l'une quelconque des revendications 1 à 8, **caractérisé en ce que** du chlorure d'hydrogène est utilisé en tant qu'halogénure d'hydrogène.

10. Procédé selon l'une quelconque des revendications 1 à 9, **caractérisé en ce que** le rapport entre le palladium et l'halogénure d'hydrogène se situe dans la plage allant de 1:3 à 1:30.

11. Procédé selon l'une quelconque des revendications 1 à 10, **caractérisé en ce qu'**un halogénure de métal est ajouté à la réaction, le rapport entre l'halogénure et l'halogénure d'hydrogène se situant dans la plage allant de 1:1 à 5:1.
